# EUROPEAN PATENT APPLICATION

(11) **EP 0 999 283 A1**
(43) Date of publication of application: **10.05.2000**
(21) Application number: 98121043.8
(22) Date of filing: 05.11.1998
(51) Int. Cl.: C12Q 1/68, A23G 1/00

(54) **Use of DNA identification techniques for the determination of genetic material of cocoa in fermented or roasted beans and chocolate**

(71) Applicant: SOCIETE DES PRODUITS NESTLE S.A., 1800 Vevey (CH)
(72) Inventor: Petiard, Vincent, 37100 Tours (FR); Crouzillat, Dominique, 37390 Cerelles (FR)
(74) Representative: Becker Kurig Straus

(57) **Abstract**

The present invention pertains to the use of DNA detection techniques for the determination of cacao in fermented and/or roasted beans and chocolate varieties and/or cacao varieties that have been modified by common breeding techniques or that have been modified by genetic engineering. In particular the DNA detection techniques are selected from the group consisting of PCR, RAPD, RFLP or microsatellite identification.

## Description

The present invention relates to the use of DNA identification techniques for the determination of genetic material of cocoa in fermented and/or roasted beans and chocolate. In particular, the present invention pertains to the use of techniques selected from the group consisting of PCR, RAPD, RFLP or microsatellite identification for the determination of cocoa varieties and/or cacao varieties that have been modified by common breeding techniques or that have been modified by genetic engineering in processed beans and chocolate.

Due to the globalisation of the world a huge variety of food products from all over the world have become available in local marketplaces. This also applies to the raw materials or intermediate products used in the various stages of food production.

Apart from new foreign food products entering local markets, novel, modified products may be obtained, that show a variety of different traits. To this end, e.g. tomato paste deriving from genetically engineered tomatoes have been sold in the United Kingdom since 1996 after having been imported from the United States of America.

Due to national regulations some food products and raw materials therefore have to be labelled so as to be able to determine their origin and/or quality level. One of the drawbacks of such labelling procedures resides in that said labels may accidentally or intentionally be exchanged so that the purchaser may not always fully rely on this sort of identification.

Therefore, there exists a need in the art for a reliable method so as to determine particular raw materials in food products of interest or their origin.

With the progress of DNA analysis techniques efficient methods for the identification of the genetic origin of raw materials/food products have become accessible. However, these techniques may only be applied to products/raw materials the genetic material of which has not been degraded to a substantial degree.

In this respect, R. Greiner at al. report in "Is there any possibility of detecting the use of genetic engineering in processed foods", Z. Ernährungswissenschaft **36** (1977), 155-160, of the option that some food products, such as pizza tomatoes, fried potatoes etc. allow for the detection of their genetic material by means of PCR (Polymerase Chain Reaction; Ehrlich et al., Science **253** (1991), 1643-1651). In the same paper (Greiner et al., supra) it is, however, also mentioned that some processed food products, such as tomato soup, tomato starch, mashed potatoes etc. do not enable such an identification, due to the DNA thereof obviously being degraded to an extensive degree.

For cocoa it was generally accepted that the process steps involved in the preparation of cocoa, such as bean fermentation, drying and roasting, which is generally performed at a temperature of 135 °C for 35 to 40 minutes, are DNA destructive processing steps such that cocoa DNA cannot be used to identify and control the genetic origin of fermented beans, roasted beans and chocolate.

Consequently, an object of the present invention resides in providing methods for the identification of the genetic material of cocoa in processed cocoa beans and chocolate.

In the studies leading to the present invention it has now surprisingly been found that contrary to the common belief of the state of the art DNA identification techniques are actually suitable to detect genetic material in processed cocoa beans and chocolate and that these techniques may efficiently be used to identify the genetic origin of cocoa.

The above object has therefore been achieved by using DNA identification techniques, in particular the techniques of PCR, RAPD, RFLP or microsatellite for the determination of genetic material of cocoa, wherein the material to be determined is fermented beans, roasted beans or chocolate.

By using the above mentioned techniques the identification of cocoa (varieties) and its (their) origin is now feasible, e.g. from which particular variety the cocoa beans contained in an end product, such as chocolate, have been derived from. This option will also represent an appealing feature so as to determine whether some particular cocoa, such as e.g. cocoa that has been modified or improved by common techniques or that has subject to genetic engineering, has been utilized in the preparation of the chocolate of interest.

Moreover, the present invention allows for an efficient control of the origin of fermented dry beans at the stage of export from/import into a particular country, for an efficient control of the origin of roasted beans in the factory during their processing and for an efficient control of chocolate on the shelf.

The techniques of PCR (Ehrlich et al., supra), RAPD (Random Amplified Polymorphic DNA; Welsh et al., "Fingerprinting genomes using PCR with arbitrary primers", Nucleic acids Res. **18** (1990), 7213-7218), RFLP (restriction fragment length polymorphism; Botstein D. et al., "Construction of a genetic map in man using restriction fragment length polymorphisms" Am. J. Hum. Genet. **32** (1980), 314-331) and microsatellite identification (Tautz, D. et al, "Hypervariability of simple sequences as a general scource for polymorphic DNA markers, Nucl. Acid. Res. **17** (1989), 6463-6471; Weber J. at al., "abundant class of human DNA polymorphisms which can be typed using the polymerase chain reaction", Am J. Hum. Genet. **44** (1989), 388-396) are known and described in the art. The above mentioned documents are incorporated herewith by way of reference.

In a preferred embodiment the DNA to be detected according to the above techniques is a DNA derived from the 5 S gene of cocoa or the SSP gene (Seed Storage Protein gene; Spencer E. et Hodge, R., Planta **186** (1992, 567-576).

In a further preferred embodiment the DNA to be detected is derived from mitochondrial and/or chloroplastic DNA. This DNA shows the advantage that it is stably propagated from mother to daughter trees thus enabling to assign the fruits of descendent trees to a particular original tree/variety. This option also applies when the descendents are derived from one female and different male trees.

By applying DNA identification techniques, preferably PCR, RAPD, RFLP or microsatellite identification techniques, with fermented and/or roasted cocoa beans and/or chocolate it will likewise be possible to identify the presence of a raw material derived from an variety, that has been modified or improved by common breeding techniques or that has been genetically modified, in the end product.

In the figures:
Fig. 1A shows a picture of an agarose gel stained with ethidium bromide, on which cocoa DNA from different scources have been applied.
Fig. 1B shows an autoradiography of a hybridisation experiment, wherein the DNA from the agarose gel of Fig. 1A has been transferred to a nylon membrane and has been hybridized with radioactively labelled cocoa DNA using ³²P as the radioactive isotope.
Fig. 2 shows a picture of an agarose gel stained with ethidium bromide, in which the 5S intergenic spacer on different cocoa samples has been amplified via PCR.
Fig. 3 shows a picture of an agarose gel stained with ethidium bromide, wherein the intron 1 and the exon 2 of the SSP gene has been amplified via PCR on different samples.
Fig. 4 shows RAPD profiles from different cocoa samples.

In the following examples the techniques for the determination of cocoa DNA in fermented and/or roasted beans and chocolate will be described. It is, however, understood that the present invention is not limited to the examples but is rather embraced by the scope of the appended claims.

Unless otherwise indicated the techniques applied are as described in Sambrook J., Fritsch E.F., Maniatis T (1989) Molecular cloning, A Laboratory Manual, second edition, Cold Spring Harbor Laboratory Press (USA).

### Example 1

### DNA extraction from fermented cocoa beans:

400 mg of cocoa embryo axes were collected, ground in liquid nitrogen and homogenised in 10 ml of extraction buffer (Sorbitol 0.35 M, Tris-HCl 0.1 M pH 8, EDTA 5 mM, Sodium bisulphite 5g/l) using an IKA-Ultra-Turrax T25 (Janke & Kunkel). The resulting homogenate is filtrated through Miracloth (Calbiochem) and centrifuged (15 min, 1000 g, 4°C). The pellet is resuspended in 5 ml of extraction buffer and centrifuged (15 min, 1000 g, 4°C). The pellet is resuspended in 0.5 ml of extraction buffer with 0.5 ml of lysis buffer (Tris-HCl 0.2 M pH 8, EDTA 50 mM, NaCl 2 M, CTAB 2%) and 0.1 ml of 5% Sarkosyl and incubated for 60 min at 65°C. The lysate was then extracted with 1 ml of chloroform/isoamyl alcohol (24/1:v/v). After centrifugation (15 min at 10.000 g, 4°C) the DNA in the aqueous phase was precipitated by adding one volume of isopropanol and standing overnight at -20°C. The DNA was collected by centrifugation, resuspended in 1 ml of TE buffer (Tris-HCl 10 mM pH 8, EDTA 0.1 mM) and purified with RNAse A (Sigma) at a final concentration of 200 µg/ml at 37°C for 30 min, and Proteinase K (Boehringer Mannheim) at a final concentration of 400 µg/ml at 56°C for 1 h. Proteins were removed with phenol/chloroform/isoamyl alcohol (25/24/1:v/v/v) treatment. After centrifugation (15 min, 1000g, 4°C) the DNA from the aqueous phase was precipitated overnight with a mixture of 3 M sodium acetate, pH 5.2, and cold ethanol (0. 1/2:v/v; -20°C). The DNA was hooked on a glass rod, washed in 70% ethanol solution and resuspended in 50 µl of TE buffer.

### Example 2

### DNA extraction from roasted cocoa beans or nibs and chocolate:

60 g of dark chocolate (Nestle, Grands chocolats, Noir (74% cocoa); Auchan, Vendome Noir (52% cocoa), freely available on the market or 25 g of nib (from Nestlé St. Menet, France) were ground in liquid nitrogen and subsequently homogenised in 200 ml of extraction buffer as previously described. The homogenate was filtrated through Miracloth and centrifuged for 15 min at 1000 g at 4°C. The pellet was resuspended in 50 ml of extraction buffer and centrifuged (15 min, 1000 g, 4°C). The pellet was resuspended in 5 ml of extraction buffer with 7 ml of lysis buffer and 2.4 ml of 5% Sarkosyl and incubated for 60 min at 65°C. The resulting lysate was then extracted with 10 ml of chloroform/isoamyl alcohol (24/1:v/v). After centrifugation (15 min, 1000 g, 4°C) the DNA in the aqueous phase was precipitated by adding one volume of isopropanol and standing overnight at -20°C. After two successive centrifugations (15 min at 10.000 g, 4°C) the resulting pellets were pooled and resuspended in 5 ml of TE buffer. Two additional purification steps with phenol/chloroform/isoamyl alcohol (25/24/1:v/v/v) were realised and the DNA from the aqueous phase finally obtained was precipitated overnight by adding 3 M sodium acetate pH 5.2 and cold ethanol (0. 1/2:v/v) and standing at -20°C. After centrifugation (15 min, 1000 g, 4°C) the DNA was resuspended in 30 µl of TE buffer. The DNA was purified with RNAse A (final concentration: 200 µg/ml) at 37°C for 10 min.

### Example 3

### PCR DNA amplification:

Two types of PCR amplification were tested, first on repeated DNA sequences from rDNA 5S gene (1000 to 50000 copies per haploid genome) and second on a single gene coding for a cocoa seed storage protein (SSP) with 3 to 5 copies per haploid genome.

### DNA amplification for the intergenic spacer of ribosomal 5S gene:

Purified DNA obtained from cocoa beans, nib or chocolate as illustrated above was diluted in TE buffer with a ratio of 1/500. The DNA amplification is performed with 5 µl of each diluted DNA samples and 45 µl of the PCR buffer containing 200 µM of each dNTP, 25 ng of each primer and 2 U of *Taq* polymerase (Stratagéne) with a final concentration of 1.5 mM magnesium chloride.

The DNA sequences of the two primers used are:
5'-TTTAGTGCTGGTATGATCGC-3' (SEQ. ID. No. I)
5'-TGGGAAGTCCTCGTGTTGCA-3' (SEQ. ID. No. II)

These two primers were designed according to Kolchinsky et al., "Portraying of plant genomes using polymerase chain reaction amplification of ribosomal 5S genes", Genome **34** (1991), 1028-1031. Amplifications were performed in a Bio-Med Thermocycler 60/2 (B. Braun) programmed for a preliminary 2-min denaturation at 94°C and 30 cycles including 1 min at 94°C, 1 min at 55°C and 1 min at 72°C. A final DNA extension cycle was performed at 72°C for 7 min.

### DNA amplification for the seed storage protein (SSP) gene:

The DNA amplification procedure was the same as previously described with the proviso that the two DNA primers designed to detect the presence of the SSP gene are as follows:
5'-GGCAATTTACTTCGTGACAAACG-3' (SEQ. ID. No. III)
5'-CTCATATTTGCCAGGAGAATTAAC-3' (SEQ. ID. No. IV)

### Example 4

### Random amplified polymorphic DNA (RAPD):

DNA amplification was performed as already described above with the proviso that the primers used are decamers randomly designed from Operon Technologies, Almeda, California.

The Codes for the corresponding primers are as follows:
AG15 5'-CCCACACGCA-3' (SEQ ID. NO V)
AM10 5'-CAGACCGACC-3' (SEQ ID NO. VI)
Z06 (to be found in the PCR kit from Operon)

Amplifications were programmed for 45 cycles including 1 min at 94°C, 1 min at 37°C and 2 min at 72°C. A final DNA extension cycle was performed at 72°C for 7 min according to Williams et al., "DNA polymorphisms amplified by arbitrary primers are useful as genetic markers", Nucl. Acids Res. **18** (1990), 6531-6535.

### DNA electrophoresis and hybridisation:

The different DNA fragments were separated on a 1.4 % agarose gel followed by staining with ethidium bromide. Southern blots were made according Southern (Sambrook, supra) using alkaline capillary transfer to nylon membranes (Appligène). The DNA probes were labelled using ³²P dCTP with the Megaprime kit (Amersham) and were hybridised on the membranes overnight at 65°C in hybridisation buffer (5% w/v SDS, 5 X SSC, 5 X Denhart's solution, 40µg/ml of heterologous DNA). Post-hybridisation treatments consisted of three high- stringency washes (2 X SSC, 0.1 % SDS; 1 X SSC, 0.1% SDS; 0.2 X SSC, 0.1% SDS) each at 65°C for 30 min.

### Example 5

### DNA detection from cocoa bean to chocolate:

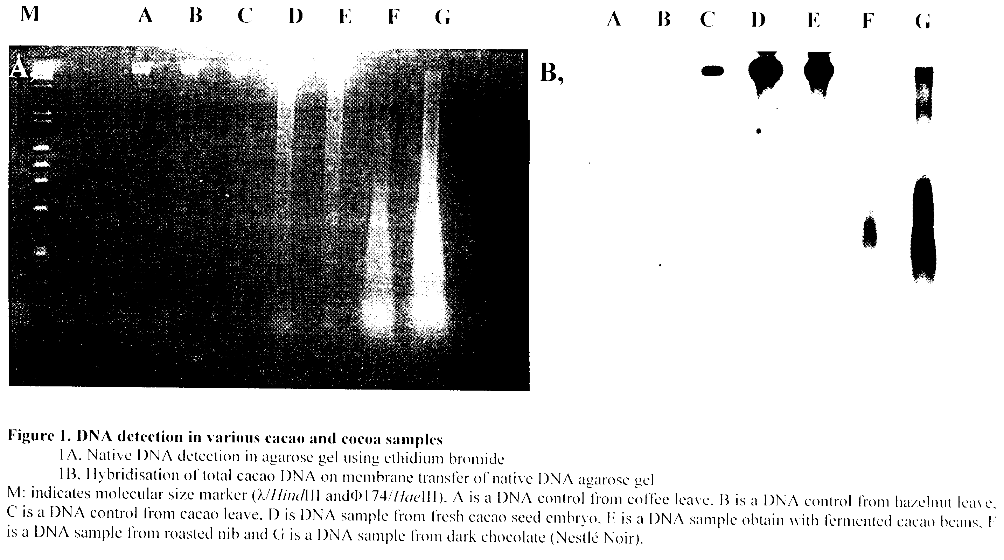

The protocol used allowed the detection of DNA of high molecular weight in fermented cocoa beans and chocolate. As negative control coffee and hazelnut DNA were used. To ensure that the DNA extracted from nib and chocolate was derived from cocoa the DNA transferred to the nylon membrane has been hybridized with radioactively labelled cocoa total DNA purified from cocoa tree leaves. The autoradiography (Figure 1B) indicates positive homology for the cocoa samples from leaves to chocolate which demonstrates that the DNA purified from these samples was originated from cocoa.

### PCR DNA amplification from cocoa samples:

In order to test the possibility to detect specific DNA sequences from the different samples specific DNA primers were used to amplify first the intergenic spacer from the 5S ribosomal gene tandemly repeated and second a part of the seed storage protein gene (SSP).

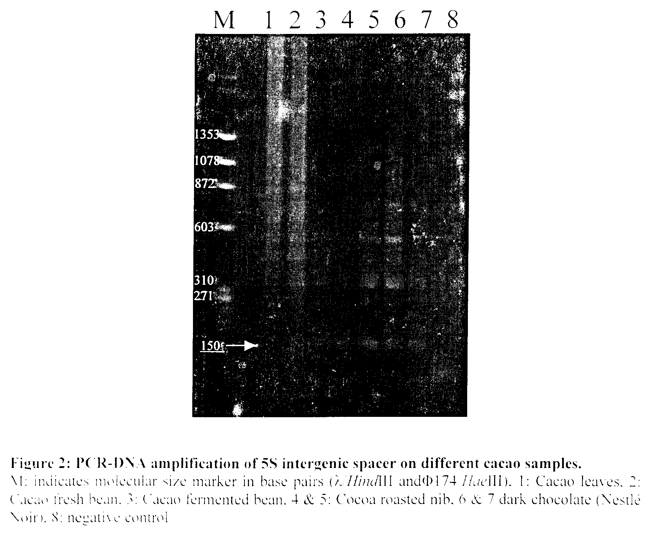

The 5S intergenic spacer amplifications were successful on all the DNA samples tested from leaves to chocolate (Figure 2). These PCR DNA amplifications were detected as a faint DNA smear indicating a substantial degradation of the DNA during the processing steps of the cocoa beans with discrete bands to be detected from approximately 160 bp to up than more 1000 bp for the leaf and fresh bean samples. These multiple DNA bands amplification were the result of the tandem repetition organisation of the target 5S ribosomal gene.

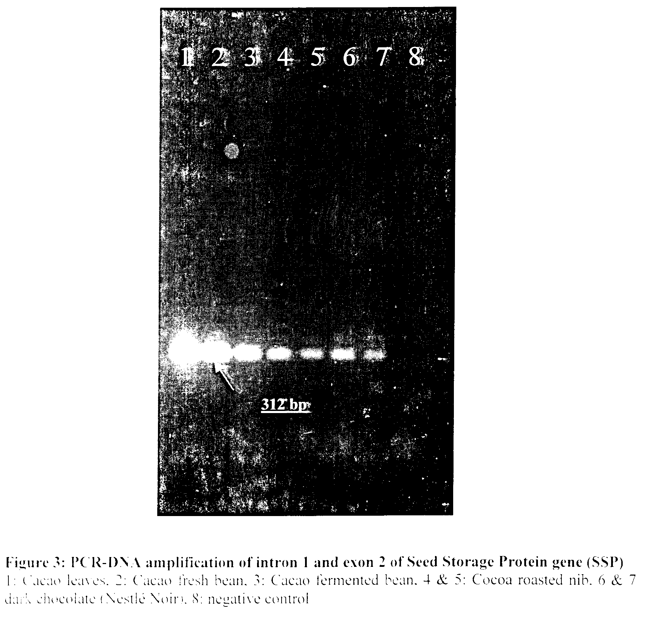

The part of the SSP gene amplified corresponds to the intron 1 and exon 2 thereof and yields a PCR product of 312 bp in length which may be detected in all the samples tested (Figure 3). This result allows to consider that it is possible to detect a single DNA gene copy from leaf to chocolate since SSP gene represents a low gene copy number in *Theobroma cocoa* haploid genome (3 to 5 copies).

DNA polymorphism was detected for the PCR DNA amplification from part of SSP gene.

### RAPD tests on cocoa samples:

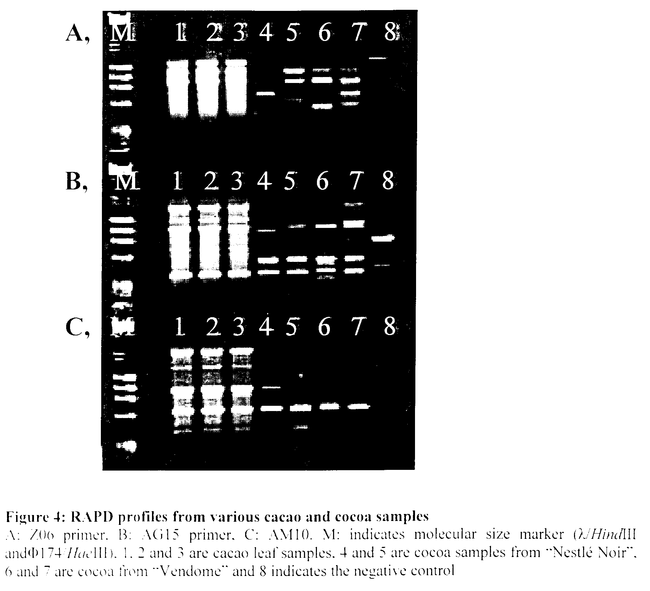

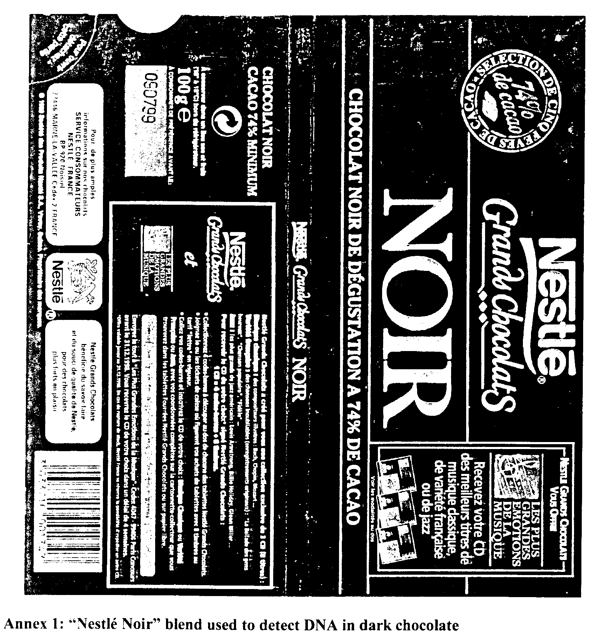

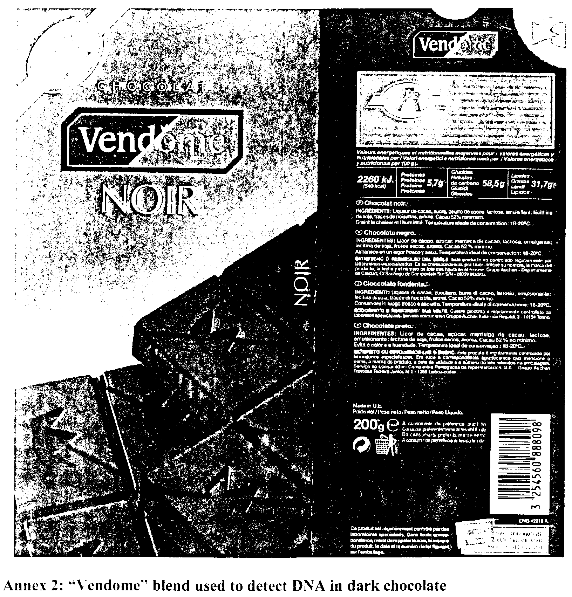

The goal of this experiment was to demonstrate that it could be possible to get RAPD fingerprint from processed cocoa samples to determine the genetic origin(s) of the raw material used for a chocolate. Three primers were selected from Operon kits and the results are illustrated by the figure 4. DNA amplifications were obtained in two different chocolate blends (Nestlé, Grands chocolats, Noir (74% cocoa); Auchan, Vendome Noir (52% cocoa)) showing that it is indeed possible to relate the end product to particular raw materials.

Sequence Listings

(1) GENERAL INFORMATION:
(i) APPLICANT: Societe des Produits Nestlé, S.A.
P.O. Box 353
CH-1800 Vevey
INVENTORS: Vincent Petiard
Dominique Crouzillat

TITEL OF THE INVENTION: Use of DNA identification techniques
for the determination of genetic material
of cocoa in fermented or roasted beans
and chocolate

(iii) NUMBER OF SEQUENCES : 6

(iv) ADDRESS:
(A) Address: Kirschner & Kurig
(B) Street: Sollnerstr. 38
(C) City: Munich
(D) State: Bavaria
(E) Country: Germany
(F) Regional Code: 81479

(v) COMPUTER READABLE FORM:
(A) Medium type: Floppy Disk
(B) Computer: IBM PC Compatibel
(C) System: PC-DOS/MS-DOS
(D) Software: Patentin Release Nr. 1, Version Nr. 1,25

(vi) INFORMATION OF THE APPLICATION:
(A) Application number: to be obtained
(B) Application date: herwewith
(C) Classification:

(vii) PRIORITY DATES: none

(viii) Attorney/Agent Information
(A) Name: Dr. Straus, Alexander
(B) Registration number: 85880
(C) Reference : E 80,022 EP

(ix) TELECOMMUNICATION:
(A) Telephone: (089) 749 858 - 0
(B) Telefax: (089) 749 858 - 11

(2) INFORMATION FOR SEQ ID. NO. 1:
(i) SEQUENCE CHARACTERISTICS:
(A) length: 20 nucleotides
(B) type: nucleic acid
(C) strandedness: single stranded
(D) topology: linear

(ii) MOLECULE TYPE: DNA

(xi) SEQENCE DESCRIPTION: SEQ ID. NO.1:

TTTAGTGCTG GTATGATCGC 20

(3) INFORMATION FOR SEQ ID. NO. 2:
(i) SEQUENCE CHARACTERISTICS:
(A) length: 20 nucleotides
(B) type: nucleic acid
(C) strandedness: single stranded
(D) topology: linear

(ii) MOLECULE TYPE: DNA

(xi) SEQENCE DESCRIPTION: SEQ ID. NO.2:

TGGGAAGTCC TCGTGTTGCA 20

(4) INFORMATION FOR SEQ ID. NO. 3:
(i) SEQUENCE CHARACTERISTICS:
(A) length: 23 nucleotides
(B) type: nucleic acid
(C) strandedness: single stranded
(D) topology: linear

(ii) MOLECULE TYPE: DNA

(xi) SEQENCE DESCRIPTION: SEQ ID. NO.3:

GGCAATTTAC TTCGTGACAA ACG 23

(5) INFORMATION FOR SEQ ID. NO. 4:
(i) SEQUENCE CHARACTERISTICS:
(A) length: 20 nucleotides
(B) type: nucleic acid
(C) strandedness: single stranded
(D) topology: linear

(ii) MOLECULE TYPE: DNA

(xi) SEQENCE DESCRIPTION: SEQ ID. NO.4:

CTCATATTTG CCAGGAGAAT TAAC 24

(6) INFORMATION FOR SEQ ID. NO. 5:
(i) SEQUENCE CHARACTERISTICS:
(A) length: 10 nucleotides
(B) type: nucleic acid
(C) strandedness: single stranded
(D) topology: linear

(ii) MOLECULE TYPE: DNA

(xi) SEQENCE DESCRIPTION: SEQ ID. NO.5:

CCCACACGCA 10

(7) INFORMATION FOR SEQ ID. NO. 6:
(i) SEQUENCE CHARACTERISTICS:
(A) length: 10 nucleotides
(B) type: nucleic acid
(C) strandedness: single stranded
(D) topology: linear

(ii) MOLECULE TYPE: DNA

(xi) SEQENCE DESCRIPTION: SEQ ID. NO.6:

CAGACCGACC 10

Sequence Listings

(1) GENERAL INFORMATION:
(i) APPLICANT: Societe des Produits Nestlé, S.A.
P.O. Box 353
CH-1800 Vevey
INVENTORS: Vincent Petiard
David Crouzillat

TITEL OF THE INVENTION: Use of DNA identification techniques
for the determination of genetic material
of cocoa in fermented or roasted beans
and chocolate

(iii) NUMBER OF SEQUENCES : 6

(iv) ADDRESS:
(A) Address: Kirschner & Kurig
(B) Street: Sollnerstr. 38
(C) City: Munich
(D) State: Bavaria
(E) Country: Germany
(F) Regional Code: 81479

(v) COMPUTER READABLE FORM:
(A) Medium type: Floppy Disk
(B) Computer: IBM PC Compatibel
(C) System: PC-DOS/MS-DOS
(D) Software: Patentin Release Nr. 1, Version Nr. 1,25

(vi) INFORMATION OF THE APPLICATION:
(A) Application number: to be obtained
(B) Application date: herwewith
(C) Classification:

(vii) PRIORITY DATES: none

(viii) Attorney/Agent Information
(A) Name: Dr. Straus, Alexander
(B) Registration number: 85880
(C) Reference : E 80,022 EP

(ix) TELECOMMUNICATION:
(A) Telephone: (089) 749 858 - 0
(B) Telefax: (089) 749 858 - 11

(2) INFORMATION FOR SEQ ID. NO. 1:
(i) SEQUENCE CHARACTERISTICS:
(A) length: 20 nucleotides
(B) type: nucleic acid
(C) strandedness: single stranded
(D) topology: linear

(ii) MOLECULE TYPE: DNA

(xi) SEQENCE DESCRIPTION: SEQ ID. NO.1:

TTTAGTGCTG GTATGATCGC 20

(3) INFORMATION FOR SEQ ID. NO. 2:
(i) SEQUENCE CHARACTERISTICS:
(A) length: 20 nucleotides
(B) type: nucleic acid
(C) strandedness: single stranded
(D) topology: linear

(ii) MOLECULE TYPE: DNA

(xi) SEQENCE DESCRIPTION: SEQ ID. NO.2:

TGGGAAGTCC TCGTGTTGCA 20

(4) INFORMATION FOR SEQ ID. NO. 3:
(i) SEQUENCE CHARACTERISTICS:
(A) length: 23 nucleotides
(B) type: nucleic acid
(C) strandedness: single stranded
(D) topology: linear

(ii) MOLECULE TYPE: DNA

(xi) SEQENCE DESCRIPTION: SEQ ID. NO.3:

(5) INFORMATION FOR SEQ ID. NO. 4:
(i) SEQUENCE CHARACTERISTICS:
(A) length: 20 nucleotides
(B) type: nucleic acid
(C) strandedness: single stranded
(D) topology: linear

(ii) MOLECULE TYPE: DNA

(xi) SEQENCE DESCRIPTION: SEQ ID. NO.4:

CTCATATTTG CCAGGAGAAT TAAC 24

(6) INFORMATION FOR SEQ ID. NO. 5:
(i) SEQUENCE CHARACTERISTICS:
(A) length: 10 nucleotides
(B) type: nucleic acid
(C) strandedness: single stranded
(D) topology: linear

(ii) MOLECULE TYPE: DNA

(xi) SEQENCE DESCRIPTION: SEQ ID. NO.5:

CCCACACGCA 10

(7) INFORMATION FOR SEQ ID. NO. 6:
(i) SEQUENCE CHARACTERISTICS:
(A) length: 10 nucleotides
(B) type: nucleic acid
(C) strandedness: single stranded
(D) topology: linear

(ii) MOLECULE TYPE: DNA

(xi) SEQENCE DESCRIPTION: SEQ ID. NO.6:

CAGACCGACC 10

## Claims

1. Use of a DNA detection technique for the determination of genetic material of cocoa in fermented and/or roasted beans and/or chocolate.

2. The use according to claim 1, wherein the DNA detection technique is selected from the group consisting of PCR, RAPD, RFLP or microsatellite identification.

3. The use according to any of the claims 1 or 2, wherein the DNA to be detected is derived from the 5 S gene of cocoa.

4. The use according to claim 1, wherein the DNA to be detected is derived from the SSP gene of cocoa.

5. The use according to claim 1, wherein the DNA to be detected is derived from mitochondrial and/or chloroplastic DNA of cocoa.

6. The use according to any of the preceding claims, wherein the cocoa from which the fermented and/or roasted beans and the chocolate are derived, is a variety, that has been modified by common breeding techniques or that has been modified by genetic engineering.
